# EUROPÄISCHE PATENTANMELDUNG

(11) **EP 3 620 181 A2**
(43) Veröffentlichungstag der Anmeldung: **11.03.2020**
(21) Anmeldenummer: 19190329.3
(22) Anmeldetag: 06.08.2019
(51) Int. Cl.: A61L 9/12, F24C 15/20

(54) **DUNSTABZUGSHAUBE**

(30) Priorität: 04.09.2018 DE 102018121517
(71) Anmelder: Miele & Cie. KG, 33332 Gütersloh (DE)
(72) Erfinder: Karsten, Olaf, 59494 Soest (DE); Hüster, Ingo, 59759 Arnsberg (DE); Wiens, Rudolf, 59494 Soest (DE)

(57) **Zusammenfassung**

Die Erfindung betrifft eine Dunstabzugshaube (1) mit wenigstens einer Beduftungseinheit (2), welche ausgebildet ist, wenigstens einen Duftstoff an die Umgebungsluft der Dunstabzugshaube (1) abzugeben. Die Dunstabzugshaube (1) ist dadurch gekennzeichnet, dass die Beduftungseinheit (2) von einer Oberseite (11) der Dunstabzugshaube (1) zugänglich angeordnet ist.

## Beschreibung

Die Erfindung betrifft eine Dunstabzugshaube gemäß des Oberbegriffs des Patentanspruchs 1.

Beispielsweise im Haushalt ist es seit Längerem bekannt, durch zusätzliche Maßnahmen die Raumluft zu verbessern. Als einfachste Maßnahme können frische Blumen aufgestellt werden, welche ihren Duft an die Umgebungsluft abgeben und so einen Raum mit einem angenehmen Geruch versehen können. Hierdurch kann nicht nur der eigene Duft der Blumen im Raum verbreitet sondern es können auch andere Düfte überdeckt werden, welche von Personen als unangenehm empfunden werden könnten. Zu diesem Zweck können beispielsweise auch parfümierte getrocknete Blumen sowie Stoffsäckchen, welche z.B. mit getrocknetem Lavendel gefüllt sind, verwendet werden.

Um den Duftstoff wirkungsvoller an die Umgebungsluft abgeben und damit einen größeren Bereich um die Duftstoffquelle herum mit dem angenehmen Geruch erfüllen zu können, ist es bekannt, künstliche Duftstoffquellen zu verwenden, welche aktiv einen Luftstrom erzeugen können. Derartige Beduftungsvorrichtungen, welche auch als Bedufter bezeichnet werden können, weisen eine Beduftungseinheit auf, welche einen Duftstoff aufnehmen, speichern und an die Umgebung aktiv abgeben kann. Üblicherweise wird ein austauschbarer Duftstoffbehälter, auch Duftstoffkapsel genannt, als Duftstoffquelle verwendet, welcher eine Duftstoffflüssigkeit aufweist.

Es kann ein Luftstrom von Umgebungsluft an der Duftstoffquelle vorbeigeführt werden, um den Duftstoff mit sich mit zu führen und an die Umgebung der Beduftungsvorrichtung abzugeben. Der Luftstrom durch die Beduftungseinheit hindurch kann durch einen Lüfter der Beduftungseinheit erfolgen, welcher Umgebungsluft durch eine Einlassöffnung ansaugen und durch eine Auslassöffnung wieder an die Umgebung abgeben kann. Derartige Beduftungsvorrichtungen sind als elektrische Raumbedufter beispielsweise von der Fa. Gries Deco Company GmbH unter dem Markenname "Air Pearls" bekannt.

Derartige Beduftungsvorrichtungen sind, wie bereits erwähnt, auch zum Überdecken von als unangenehm empfundenen Gerüchen einsetzbar. Dies kann beispielsweise in einer Küche der Fall sein, um die bei der Zubereitung der Speisen und insbesondere beim Braten entstehenden Gerüche zu überdecken. Hierzu können insbesondere Düfte bzw. Duftnoten verwendet werden, welche in eine Küchenumgebung passen aber als angenehm empfunden werden wie beispielsweise der Duft verschiedener Kräuter. Entsprechend ist von der Fa. Elica S.P.A. ein Duftzerstäuber "Marie" bekannt, welcher speziell für den Einsatz in Küchen gedacht ist, um Küchengerüche zu neutralisieren. Diese Beduftungsvorrichtung kann somit als Küchengerät angesehen werden.

Nachteilig ist bei derartigen Beduftungsvorrichtungen, dass diese nur unzureichend dort eingesetzt werden können, wo die als unangenehm empfindbaren Küchengerüche entstehen, und zwar in der unmittelbaren Umgebung des Kochfelds. Derartige Beduftungsvorrichtungen sind nämlich als einzelne Standgeräte dazu gedacht, dort auf einer Oberfläche wie z.B. auf der Arbeitsplatte oder auf einem Regalboden einer Küchenzeile aufgestellt zu werden, wo sie betrieben werden sollen. Um somit den Geruch einer Benutzung z.B. einer Bratpfanne zu neutralisieren und zu überdecken, ist die Beduftungsvorrichtung entsprechend nah neben dem Kochfeld bzw. der Kochstelle anzuordnen. Dies kann jedoch bereits zu weit von der Bratpfanne entfernt sein, um wirkungsvoll betrieben werden zu können. Insbesondere können die zu neutralisierenden Bratgerüche nach oben und über die Beduftungsvorrichtung hinweg aufsteigen, so dass die Bratgerüche gar nicht von dem Duftstoff der Beduftungsvorrichtung erreicht und somit auch nicht neutralisiert werden können.

Nachteilig ist bei einer derartigen Anwendung auch, dass die Beduftungsvorrichtung z.B. von Fettspritzern getroffen und hierdurch schmierig verschmutzt werden kann. Dies führt zu einem entsprechenden Reinigungsaufwand durch den Benutzer. Ggfs. kann das Fett sogar durch die Auslassöffnung des Luftstroms in das Innere der Beduftungsvorrichtung gelangen, was zum einen die Reinigung aufwendiger bis unmöglich machen sowie die Beduftungsvorrichtung in ihrer Funktion einschränken kann.

Nachteilig ist ferner, dass derartige Beduftungsvorrichtungen zwecks Versorgung mit elektrischer Energie netzgebunden über ein Kabel mit einer Steckdose verbunden oder kabellos mittels elektrischer Energiespeicher betrieben werden müssen. Wird die Beduftungsvorrichtung an eine Steckdose angeschlossen, schränkt dies die Einsatzmöglichkeiten auf den Bereich ein, welcher aufgrund der Länge des Stromkabels erreicht werden kann. Auch kann das Stromkabel der Beduftungsvorrichtung vom Benutzer als störend empfunden werden, weil ggfs. die Arbeitsplatte in der Benutzung hierdurch eingeschränkt werden kann. Alternativ wiederaufladbare oder einmal nutzbare elektrische Energiespeicher wie z.B. Akkumulatoren bzw. Batterien zu verwenden kann dazu führen, dass eine Benutzung der Beduftungsvorrichtung mit entleertem Energiespeicher nicht möglich ist. Somit muss der Energiespeicher regelmäßig aufgeladen oder ausgetauscht werden, was einen zusätzlichen Aufwand für den Benutzer darstellt.

Nachteilig ist auch, dass eine derartige Beduftungsvorrichtung, insbesondere als stromkabellose Beduftungsvorrichtung, vom Benutzer aufgestellt werden muss, um verwendet werden zu können. Insbesondere ist der Einsatzort zu wählen. Dabei ist darauf zu achten, dass die Beduftungsvorrichtung nicht anderen Küchengeräten, Kochutensilien und dergleichen im Wege ist und Platz auf der Arbeitsfläche der Küchenzeile einnimmt, welcher dann nicht mehr anderweitig genutzt werden kann. Auch kann, andersherum betrachtet, nur eine eingeschränkte Anzahl von Einsatzorten für die Beduftungsvorrichtung zur Verfügung stehen, falls zuerst die übrigen Küchengeräte, Kochutensilien und dergleichen vom Benutzer aufgestellt werden. Diese eingeschränkte Möglichkeit von Einsatzorten für die Beduftungsvorrichtung kann deren wirkungsvollen Einsatz widersprechen, so dass die Beduftungsvorrichtung ihre Aufgabe nur unzureichend bis gar nicht erfüllen kann. Auch ist die Beduftungsvorrichtung bei Nichtbenutzung wegzuräumen, was ebenfalls einen zusätzlichen Aufwand für den Benutzer bedeutet.

Nachteilig ist ebenso, dass eine stromkabellose Beduftungsvorrichtung beim Wegräumen oder auch beim Verändern ihres Einsatzortes derart vom Benutzer räumlich verändern werden kann, dass der Benutzer die Beduftungsvorrichtung bei der nächsten Benutzung gar nicht mehr oder lediglich nach längerem Suchen wiederfinden kann. Dies kann die Benutzung verhindern, verzögern oder den Benutzer sogar davon abhalten, die Beduftungsvorrichtung einzusetzen.

Nachteilig ist auch, dass die Beduftungsvorrichtung vom Benutzer zu bedienen ist, da üblicherweise nicht kontinuierlich Duftstoff an die Umgebung abgegeben wird sondern lediglich im Bedarfsfall. Andernfalls wäre die Duftstoffquelle zu schnell entleert und bzw. oder die Duftstoffkonzentration könnte zu hoch und vom Benutzer als unangenehm empfunden werden. Dieser Bedarfsfall muss üblicherweise der Beduftungsvorrichtung vom Benutzer mitgeteilt werden, indem z.B. über eine Betätigung einer Aktivierungsfunktion wie z.B. mittels eines Tasters oder dergleichen ein Auslösen der Duftabgabe der Beduftungseinheit der Beduftungsvorrichtung vom Benutzer veranlasst wird. Somit muss der Benutzer aktiv tätig werden, um die Funktion der Beduftungsvorrichtung nutzen zu können. Dies stellt einen zusätzlichen Aufwand für den Benutzer dar. Wird dabei das Auslösen der Duftabgabe vom Benutzer vergessen oder zu spät durchgeführt, beispielsweise erst im Laufe einer Zubereitung nachdem bereits unangenehme Gerüche entstanden sind, so kann die Beduftungsvorrichtung gar nicht oder zumindest nicht wirkungsvoll ihre Aufgabe erfüllen.

Zusammenfassend sind somit zwar Beduftungsvorrichtungen insbesondere für die Verwendung in einer Küche bekannt. Jedoch stellt sich die Verwendung bisher recht umständlich für den Benutzer dar. Auch kann die Verwendung der Küche bzw. der übrigen Küchengeräte hierdurch eingeschränkt werden. Insbesondere kann die Duftabgabe nicht sicher dazu führen, dass die zu überdeckenden Küchengerüche auch tatsächlich von dem Duftstoff der Beduftungsvorrichtung neutralisiert werden.

Der Erfindung stellt sich somit das Problem, eine Dunstabzugshaube als Küchengerät der eingangs beschriebenen Art und Weise bereitzustellen, so dass die zuvor beschriebenen Nachteile überwunden werden können. Insbesondere soll eine Dunstabzugshaube zur Duftabgabe und bzw. oder zur Neutralisierung von Küchengerüchen bereitgestellt werden, welches seine Funktion einfacher und bzw. oder verlässlicher als bisher bekannt ausüben kann. Zumindest soll eine Alternative zu bekannten derartigen Dunstabzugshauben bereitgestellt werden.

Erfindungsgemäß wird dieses Problem durch eine Dunstabzugshaube mit den Merkmalen des Patentanspruchs 1 gelöst. Vorteilhafte Ausgestaltungen und Weiterbildungen der Erfindung ergeben sich aus den nachfolgenden Unteransprüchen.

Somit betrifft die vorliegende Erfindung eine Dunstabzugshaube mit wenigstens einer Beduftungseinheit, welche ausgebildet ist, wenigstens einen Duftstoff an die Umgebungsluft der Dunstabzugshaube abzugeben. Die Dunstabzugshaube ist dadurch gekennzeichnet, dass die Beduftungseinheit von einer Oberseite der Dunstabzugshaube zugänglich angeordnet ist.

Der vorliegenden Erfindung liegt dabei die Erkenntnis zugrunde, dass die eingangs genannten Nachteile teilweise oder vollständig dadurch behoben werden können, dass eine Beduftungseinheit direkt in eine Dunstabzugshaube integriert und von der Oberseite der Dunstabzugshaube zugänglich angeordnet wird. Auf diese Art und Weise muss die Beduftungseinheit bzw. eine Beduftungsvorrichtung weder aufgestellt noch nach der Benutzung weggeräumt werden. Auch kann ein zusätzlicher Platzbedarf für eine separate Beduftungsvorrichtung vermieden werden. Dies kann die Küchenzeile frei von einer zusätzlichen Beduftungsvorrichtung halten sowie Stauraum sparen. Auch kann auf diese Art und Weise sichergestellt werden, dass die Beduftungseinheit dem Benutzer jederzeit zur Verfügung stehen kann. Ebenso muss die Beduftungseinheit nicht gesucht und aufgestellt werden, um betrieben werden zu können. Auch kann dies bei jeglicher Art von Dunstabzugshauben den Zugriff für den Benutzer erleichtern. Wird insbesondere eine Dunstabzugshaube verwendet, welche oberhalb eines Kochfelds aufgehängt ist, so kann auf diese Art und Weise die Beduftungseinheit aus dem Blickfeld des Benutzers ferngehalten werden. Es können sich durch die Integration einer Beduftungseinheit in eine Dunstabzugshaube weitere Vorteile ergeben, wie weiter unten näher beschrieben wird.

Gemäß einem Aspekt der vorliegenden Erfindung ist die Beduftungseinheit, vorzugsweise ein Lüfter der Beduftungseinheit, ausgebildet, vom Benutzer mittels wenigstens eines Bedienelements der Dunstabzugshaube bedient zu werden. Vorzugsweise ist der Lüfter motorisch angetrieben und/oder rotarorisch beweglich, insbesondere als Luftstromerzeuger ausgebildet. Auf diese Art und Weise kann auf eine separate und zusätzliche Anordnung eines Bedienelements extra für die Beduftungseinheit verzichtet werden, indem die Bedienelemente der Dunstabzugshaube mitverwendet werden. Dies kann auch die Benutzung der Beduftungseinheit für den Benutzer intuitiver gestalten. Dies gilt entsprechend für eine Steuerung bzw.

Elektronik der Dunstabzugshaube, welche ebenfalls den Lüfter mit ansteuern und kontrollieren kann.

Gemäß einem weiteren Aspekt der vorliegenden Erfindung ist die Beduftungseinheit, vorzugsweise ein Lüfter der Beduftungseinheit, ausgebildet, von der Dunstabzugshaube elektrisch versorgt zu werden. Auf diese Art und Weise kann auf eine zusätzliche elektrische Versorgung der Beduftungseinheit verzichtet werden. Dies kann den Aufwand und auch die Kosten der Verkabelung geringer halten.

Gemäß einem weiteren Aspekt der vorliegenden Erfindung weist die Beduftungseinheit wenigstens einen Duftstoffquellenhalter auf, welcher ausgebildet ist, wenigstens eine Duftstoffquelle austauschbar aufzunehmen und in der Beduftungseinheit zu halten. Eine Duftstoffquelle kann insbesondere ein Duftstoffbehälter wie eine Duftstoffkapsel sein, welche auch als Duftflakon bezeichnet werden kann. Eine derartige Duftstoffquelle kann von dem Duftstoffquellenhalter aufgenommen werden, damit die Duftstoffquelle samt dem Duftstoffquellenhalter vom Benutzer gemeinsam gehandhabt werden kann. Dies kann die Handhabung für den Benutzer vereinfachen. Auch kann die Duftstoffquelle mittels des Duftstoffquellenhalters definiert in der Beduftungseinheit angeordnet werden, um dort betrieben zu werden. Dies kann auch ein unerwünschtes Auslaufen einer Duftstoffflüssigkeit aus der Duftstoffquelle in den Duftstoffquellenhalter bzw. in die Beduftungseinheit vermeiden helfen.

Die Duftstoffquelle weist vorzugsweise eine Außenkontur auf, welche in keiner Raumrichtung eine größere Ausdehnung als ca. 20 cm aufweist. Vorzugsweise weist die Duftstoffquelle in jeder Raumrichtung eine Ausdehnung von weniger als ca. 10 cm und größer als ca. 1 cm auf. Die Duftstoffquelle weist üblicherweise eine weitestgehend kubische oder zylindrische Form auf, wobei die Duftstoffquelle vorzugsweise in zwei Raumrichtungen näherungsweise dieselbe Ausdehnung im Kreisquerschnitt oder im Quadratquerschnitt aufweist und in die dritte Raumrichtung, gegenüber den beiden anderen Raumrichtungen, eine abweichende, entweder kleinere oder größere, Ausdehnung aufweist, welche vorzugsweise um den Faktor ca. 1,5 abweicht. Hierdurch kann eine besonders gute Handhabbarkeit, insbesondere beim Einsetzen der Duftstoffquelle in den Duftstoffquellenhalter, erreicht werden.

Gemäß einem weiteren Aspekt der vorliegenden Erfindung ist der Duftstoffquellenhalter ausgebildet, von der Beduftungseinheit entnehmbar aufgenommen zu werden. Mit anderen Worten kann der Duftstoffquellenhalter aus der Beduftungseinheit entfernt werden, um insbesondere die Duftstoffquelle auszutauschen. Dies kann erforderlich sein, wenn der Duftstoff der Duftstoffquelle aufgebraucht ist. Wurde die Duftstoffquelle erneuert, so kann der Duftstoffquellenhalter samt der Duftstoffquelle von der Beduftungseinheit wieder aufgenommen werden, damit die Beduftungseinheit wieder betrieben werden kann.

Gemäß einem weiteren Aspekt der vorliegenden Erfindung weist die Beduftungseinheit einen Luftführungsschacht auf, welcher ausgebildet ist, Umgebungsluft der Dunstabzugshaube zugeführt zu bekommen, durch die Beduftungseinheit hindurchzuführen und über wenigstens eine Auslassöffnung, vorzugsweise über eine Mehrzahl von Auslassöffnungen, an die Umgebung der Dunstabzugshaube wieder abzugeben, wobei der Duftstoffquellenhalter ausgebildet ist, in dem Luftführungsschacht aufgenommen zu werden. Mittels dieses Luftführungsschachts kann ein Luftstrom durch die Beduftungseinheit hindurchgeführt werden, um den Duftstoff der Duftstoffquelle aufzunehmen und an die Umgebung abzugeben.

Hierzu kann zum einen ein Teil eines Luftstroms verwendet werden, welcher von dem Luftstrom der Dunstabzugshaube abgezweigt wird. Zu diesem Zweck kann beispielsweise in der Dunstabzugshaube eine entsprechende Verzweigung des Luftstroms ausgebildet sein, so dass über einen zusätzlichen Kanal in Form des Luftführungsschachts ein Teil der ohnehin vom Hauptgebläse der Dunstabzugshaube angesaugten Umgebungsluft für die Anreicherung mit dem Duftstoff verwendet werden kann. Es ist jedoch auch möglich, einen separaten Luftstrom zu erzeugen, welcher lediglich durch den Luftführungsschacht geführt wird und der Anreicherung des Luftstroms mit dem Duftstoff dient.

Vorzugsweise wird die Auslassöffnung von dem Duftstoffquellenhalter ausgebildet. Auf diese Art und Weise kann die Funktion der Auslassöffnung direkt in den Duftstoffquellenhalter, z.B. als Auslassschlitze, integriert werden. Dies kann die Konstruktion der Beduftungseinheit vereinfachen.

Um die optimale Beduftung zu erzielen, können vorzugsweise die folgenden Maße bzw. Verhältnisse des Luftführungsschachts verwendet werden. Der kleinste Schachtquerschnitt mit ca. 21,5 cm² minus dem größtem Duftstoffquellenquerschnitt mit ca. 13 cm² ergibt einen Durchströmungsquerschnitt von ca. 11,5 cm² im Verhältnis zur größten Abdampffläche (Dochtfläche) der Duftstoffquelle mit 3,3 cm². Die Ausblasfläche steht wie folgt im Verhältnis: Der kleinste Schachtquerschnitt mit ca. 21,5 cm² minus dem größtem Duftstoffquellenquerschnitt mit ca. 13 cm² ergibt einen Durchströmungsquerschnitt von ca. 11,5 cm² im Verhältnis zur größten Ausblasöffnung von 6,5 cm².

Gemäß einem weiteren Aspekt der vorliegenden Erfindung weist die Beduftungseinheit wenigstens eine Halterungseinheit auf, welche ausgebildet ist, den aufgenommenen Duftstoffquellenhalter in der Beduftungseinheit zu halten. Auf diese Art und Weise kann der Duftstoffquellenhalter nicht nur lose in die Beduftungseinheit eingesetzt werden, sondern mittels der Halterungseinheit auch dort gehalten werden. Dies kann eine sichere Verwendung des Duftstoffquellenhalters ermöglichen. Insbesondere kann ein Auslaufen von Duftstoffflüssigkeit aus der von dem Duftstoffquellenhalter aufgenommenen Duftstoffquelle verhindern.

Gemäß einem weiteren Aspekt der vorliegenden Erfindung wird die Halterungseinheit von wenigstens einer Aufnahme der Beduftungseinheit, vorzugsweise von wenigstens einer Aufnahme eines Gehäuses der Beduftungseinheit, aufgenommen. Hierdurch kann die Halterungseinheit einfach von der Beduftungseinheit gehalten werden, was die Konstruktion sowie die Montage einfach und kostengünstig werden lassen kann.

Vorzugsweise weist das Gehäuse wenigstens zwei Gehäuseelemente, vorzugsweise genau zwei Gehäuseelemente auf, welche gemeinsam die Aufnahme ausbilden und die Halterungseinheit zwischen sich aufnehmen. Dies kann eine möglichst einfache Ausbildung der beiden Gehäuseelemente, z.B. als Spritzgussteile, ermöglichen, so dass die zuvor beschriebene Funktion möglichst einfach realisiert werden kann.

Gemäß einem weiteren Aspekt der vorliegenden Erfindung ist die Halterungseinheit als Push-Push-Verriegelungsmechanismus ausgebildet und der Duftstoffquellenhalter weist, vorzugsweise an seiner Unterseite, wenigstens ein Rastelement auf, welches ausgebildet ist, von dem Push-Push-Verriegelungsmechanismus gehalten zu werden. Somit kann es ermöglicht werden, dass ein Benutzer den Duftstoffquellenhalter in die Beduftungseinheit einführen und durch Herunterdrücken dort einrastend verriegeln kann. Um den Duftstoffquellenhalter wieder aus der Beduftungseinheit entnehmen zu können, kann der Benutzer erneut den Push-Push-Verriegelungsmechanismus durch Drücken betätigen, so dass der Duftstoffquellenhalter aus der Beduftungseinheit herausgedrückt wird. Hierdurch kann der Duftstoffquellenhalter für den Benutzer einfacher mit den Fingern zu greifen und zu entnehmen sein.

Vorzugsweise weist der Duftstoffquellenhalter, vorzugsweise an seiner Oberseite, wenigstens ein Betätigungselement, vorzugsweise wenigstens eine Fingermulde, auf, welche ausgebildet ist, durch Betätigung durch einen Benutzer den Push-Push-Verriegelungsmechanismus zu betätigen. Auf diese Art und Weise kann dem Benutzer haptisch eine Information gegeben werden, an welcher Stelle er den Push-Push-Verriegelungsmechanismus zu betätigen hat, um die Duftstoffquellenhalter einzurasten oder zu lösen. Dies kann insbesondere dann nützlich sein, wenn die Beduftungseinheit derart von der Dunstabzugshaube aufgenommen wird, dass diese für den Benutzer optisch nicht zu erfassen ist.

Gemäß einem weiteren Aspekt der vorliegenden Erfindung weist die Beduftungseinheit, vorzugsweise ein Gehäuse der Beduftungseinheit, wenigstens ein Führungselement auf, welches ausgebildet ist, den Duftstoffquellenhalter beim Entnehmen und bzw. oder beim Einsetzen zu führen, wobei der Duftstoffquellenhalter wenigstens eine Aufnahme für wenigstens ein korrespondierendes Führungselement aufweist. Dies kann die Bewegung des Duftstoffquellenhalters innerhalb der Beduftungseinheit vereinfachen, weil diese mechanisch geführt ablaufen kann. Dies kann sowohl für das Entnehmen als auch für das Einsetzen des Duftstoffquellenhalters in der Beduftungseinheit gelten.

Hierbei ein entsprechendes Führungselement lediglich auf einer Seite des Duftstoffquellenhalters und korrespondierend auf der Seite der Beduftungseinheit vorzusehen kann gleichzeitig eine Codierung ermöglichen, da der Duftstoffquellenhalter lediglich in einer Orientierung in die Beduftungseinheit eingesetzt werden kann. Dies kann sicherstellen, dass der Duftstoffquellenhalter vom Benutzer bestimmungsgemäß in die Beduftungseinheit eingesetzt wird.

Vorzugsweise weist die Beduftungseinheit, vorzugsweise das Gehäuse der Beduftungseinheit, wenigstens einen Führungszapfen als Führungselement und bzw. oder wenigstens eine Führungsschiene als Führungselement auf. Ein derartiger Führungszapfen mit einer korrespondierenden Nut des Duftstoffquellenhalters kann insbesondere das Einsetzen vereinfachen sowie eine Codierung der Orientierung des Duftstoffquellenhalters beim Einsetzen, wie zuvor beschrieben, realisieren. Mittels einer Führungsschiene und einer korrespondierenden Aufnahme des Duftstoffquellenhalters kann eine Bewegung geführt ablaufen.

Gemäß einem Aspekt der vorliegenden Erfindung weist die Dunstabzugshaube wenigstens einen Lüfter auf, welcher ausgebildet und angeordnet ist, einen Luftstrom durch die Beduftungseinheit, vorzugsweise durch eine von einem Duftstoffquellenhalter aufgenommene Duftstoffquelle, zu erzeugen, wobei der Lüfter vorzugsweise ein zusätzlicher Lüfter der Beduftungseinheit ist und die Dunstabzugshaube einen Hauptlüfter aufweist. Hierdurch kann ein aktiver Luftstrom erzeugt werden, um entsprechend viel Duftstoff aus der Duftstoffquelle an die Luft, welche die Beduftungseinheit durchströmt, abzugeben. Die Duftstoffquelle kann in Abhängigkeit der Stärke des Luftstroms entsprechend wenig bzw. viel Duftstoff an die Umgebung abgeben.

Gemäß einer Ausführungsform ist der Lüfter der Beduftungseinheit als ein zusätzlicher Lüfter vorgesehen, ergänzend zu einem Hauptlüfter welchen die Dunstabzugshaube aufweist. Hierdurch kann der Luftstrom der Beduftungseinheit unabhängig von dem Betrieb der Dunstabzugshaube oder des Hauptlüfters erzeugt werden.

Vorzugsweise kann dabei die Drehzahl des Lüfters, welcher auch als Gebläse bezeichnet werden kann, durch den Benutzer eingestellt werden. Dies kann stufenlos oder in diskreten vorbestimmten Schritten erfolgen. Hierdurch kann über die Stärke des Luftstroms bzw. über die Menge der zugeführten Umgebungsluft auch die Menge von Duftstoff, welche an die Umgebung abgegeben wird, durch den Benutzer beeinflusst werden.

Als Lüfter kann grundsätzlich jeglicher Lüfter verwendet werden, welche in der Lage ist, den geforderten Luftstrom durch die Beduftungseinheit hindurch zu erzeugen. Beispielsweise kann ein Teil des Luftstroms eines Hauptlüfters der Dunstabzugshaube abgezweigt und durch die Beduftungseinheit hindurch geführt werden, so dass auf einen zusätzlichen Lüfter hierfür verzichtet werden kann. Dies kann Kosten, Montageaufwand und bzw. oder Bauraum sparen helfen.

Vorzugsweise ist der Lüfter ein zusätzlicher Lüfter der Beduftungseinheit und die Dunstabzugshaube weist einen Hauptlüfter auf. Mit anderen Worten wird die Funktion der Luftförderung der Dunstabzugshaube an sich unverändert gelassen, so dass diese unverändert ausgelegt und umgesetzt werden kann. Vielmehr wird ein zusätzlicher Lüfter nur für die Beduftungseinheit vorgesehen, welcher den Luftstrom durch die Beduftungseinheit hindurch erzeugen kann. Hierdurch kann ein unabhängiger Luftstrom erzeugt werden, welcher lediglich diesem Zweck dient und entsprechend vom Benutzer eingestellt werden kann. Insbesondere kann die Beduftungseinheit unabhängig von der Hauptfunktion der Dunstabzugshaube, Gargerüche zu filtern, betrieben werden, da nicht mit jedem Garvorgang Gerüche entstehen, welche mittels der Beduftungseinheit überdeckt werden müssen.

Gemäß einem weiteren Aspekt der vorliegenden Erfindung ist der Lüfter innerhalb eines Gehäuses der Beduftungseinheit angeordnet. Auf diese Art und Weise kann der Lüfter von dem Gehäuse der Beduftungseinheit gehalten und sicher positioniert werden. Auf entsprechende zusätzliche Halterungsmittel kann verzichtet werden, was die Konstruktion des Duftstoffquellenhalters vereinfachen und kostengünstiger werden lassen kann.

Vorzugsweise wird der Lüfter in der Strömungsrichtung des Luftstroms wenigstens einseitig von einer Lüfteraufnahme des Gehäuses gehalten. Dies kann den sicheren Halt ermöglichen, indem die einseitige Lüfteraufnahme vorzugsweise unterhalb des Lüfters angeordnet ist, so dass dieser mittels Schwerkraft auf der Lüfteraufnahme aufliegen und von dieser gehalten werden kann.

Vorzugsweise wird der Lüfter in der Strömungsrichtung des Luftstroms beidseitig von einer ersten Aufnahmekante des Gehäuses und von einer zweiten Aufnahmekante des Gehäuses gehalten. Hierdurch kann eine beidseitige Abstützung bzw. Halterung des Lüfters erfolgen. Dies kann bei einer vertikalen Strömungsrichtung des Luftstroms sowohl von unten als auch von oben erfolgen, so dass der Lüfter im Betrieb bei entsprechend enger Bemaßung der beiden Aufnahmekanten nicht schwingen kann. Bei einer horizontalen Ausrichtung der Strömungsrichtung des Luftstroms kann der Lüfter durch die beidseitige Halterung ferner davon abgehalten werden, seitlich zu kippen.

Alternativ könnte der Lüfter auch durch Verschraubung, durch Verklebung oder durch Vernietung mit dem Gehäuse des Beduftungseinheit verbunden und von diesem gehalten werden. Dies könnten jedoch einen zusätzlichen Aufwand an Material sowie an Montageaufwand bedeuten, so dass die zuvor beschriebene Art der Halterung aufgrund ihrer Einfachheit zu bevorzugen sein kann, da auf zusätzliche Befestigungsmittel verzichtet werden kann.

Gemäß einem weiteren Aspekt der vorliegenden Erfindung weist die Beduftungseinheit ein Gehäuse mit wenigstens zwei Gehäuseelementen, vorzugsweise mit genau zwei Gehäuseelementen, auf, wobei die beiden Gehäuseelemente jeweils Verbindungselemente aufweisen, welche ausgebildet sind, die beiden Gehäuseelemente formschlüssig und bzw. oder kraftschlüssig miteinander zu verbinden. Als Verbindungselemente können jegliche mechanische Mechanismen verwendet werden, welche in der Lage sind, eine entsprechende formschlüssige und bzw. oder kraftschlüssige Verbindung der beiden Gehäuseelemente miteinander zu erreichen.

Vorzugsweise weist das erste Gehäuseelement wenigstens ein Rastelement, vorzugsweise eine Mehrzahl von Rastelementen, als Verbindungselement und das zweite Gehäuseelement wenigstens ein Hakenelement, vorzugsweise eine Mehrzahl von Hakenelementen, als Verbindungselement auf, welche ausgebildet sind, die beiden Gehäuseelemente formschlüssig und bzw. oder kraftschlüssig miteinander zu verbinden. Auf diese Art und Weise kann ein einfacher Rastmechanismus erreicht werden, welcher einen sicheren Halt ermöglichen kann. Auch kann dieser gegebenenfalls durch Aufbiegen der Rastelemente bzw. Hakenelemente wieder zerstörungsfrei geöffnet werden, um gegebenenfalls das Innere des Gehäuses z.B. zum Austausch eines defekten Lüfters erreichen zu können. Hierbei mehrere Rastelemente sowie Hakenelemente zu verwenden, kann dahingehend vorteilhaft sein, dass sie im Zusammenwirken eine sichere Verbindung erreichen können als lediglich ein einzelnes Verbindungselement. Insbesondere können derartige Verbindungselemente bei Spritzgussteilen einfach einstückig mit dem jeweiligen Gehäuseelement ausgebildet werden.

Gemäß einem weiteren Aspekt der vorliegenden Erfindung weist die Beduftungseinheit, vorzugsweise ein Gehäuse der Beduftungseinheit, wenigstens eine Befestigungsmittelaufnahme, vorzugsweise eine Mehrzahl von Befestigungsmittelaufnahmen, auf, welches ausgebildet ist, wenigstens ein Befestigungsmittel aufzunehmen, so dass die Beduftungseinheit von der Dunstabzugshaube gehalten werden kann.

Gemäß einem weiteren Aspekt der vorliegenden Erfindung weist die Beduftungseinheit, vorzugsweise ein Gehäuse der Beduftungseinheit, wenigstens ein Halterungselement, vorzugsweise eine Mehrzahl von Halterungselementen, auf, welches ausgebildet ist, so dass die Beduftungseinheit von der Dunstabzugshaube gehalten werden kann.

Im ersteren Fall kann durch die Befestigungsmittelaufnahme ein Befestigungsmittel der Dunstabzugshaube aufgenommen werden. Als Befestigungsmittel der Dunstabzugshaube können beispielsweise Schrauben verwendet werden, welche durch eine Durchgangsöffnung eines Gehäuses der Dunstabzugshaube hindurch in die Befestigungsmittelaufnahme der Beduftungseinheit eingreifen und diese dadurch halten können. Dies kann eine sichere und dauerhafte Montage der Beduftungseinheit in der Dunstabzugshaube ermöglichen. Alternativ könnte die Befestigung der Beduftungseinheit in der Dunstabzugshaube auch mittels Vernieten, mittels Verkleben, mittels Verrasten oder dergleichen erfolgen.

In dem zweiten Fall kann ein Halterungselement der Beduftungseinheit in eine entsprechende Aufnahme der Dunstabzugshaube eingreifen, um zumindest einen vorläufigen Halt bei der Montage zu ermöglichen. In diesem Zustand kann die zuvor beschriebene Befestigung, z.B. mittels Schrauben, bei der Montage vorgenommen werden, ohne dass die Beduftungseinheit hierdurch von einer Person gehalten werden muss. Dies kann die Montage vereinfachen.

Ein Ausführungsbeispiel der Erfindung ist in den Zeichnungen rein schematisch dargestellt und wird nachfolgend näher beschrieben. Es zeigt
- Figur 1: eine perspektivische Darstellung einer erfindungsgemäßen Dunstabzugshaube;
- Figur 2: eine Detailansicht der Figur 1;
- Figur 3: einen Querschnitt einer Detailansicht der Figur 2;
- Figur 4: eine Explosionszeichnung der Beduftungseinheit der erfindungsgemäßen Dunstabzugshaube;
- Figur 5: einen Querschnitt der Beduftungseinheit der erfindungsgemäßen Dunstabzugshaube; und
- Figur 6: eine perspektivische Darstellung der Beduftungseinheit der erfindungsgemäßen Dunstabzugshaube.

Die o.g. Figuren werden in kartesischen Koordinaten betrachtet. Es erstreckt sich eine Längsrichtung X, welche auch als Tiefe X bezeichnet werden kann. Senkrecht zur Längsrichtung X erstreckt sich eine Querrichtung Y, welche auch als Breite Y bezeichnet werden kann. Senkrecht sowohl zur Längsrichtung X als auch zur Querrichtung Y erstreckt sich eine vertikale Richtung Z, welche auch als Höhe Z bezeichnet werden kann.

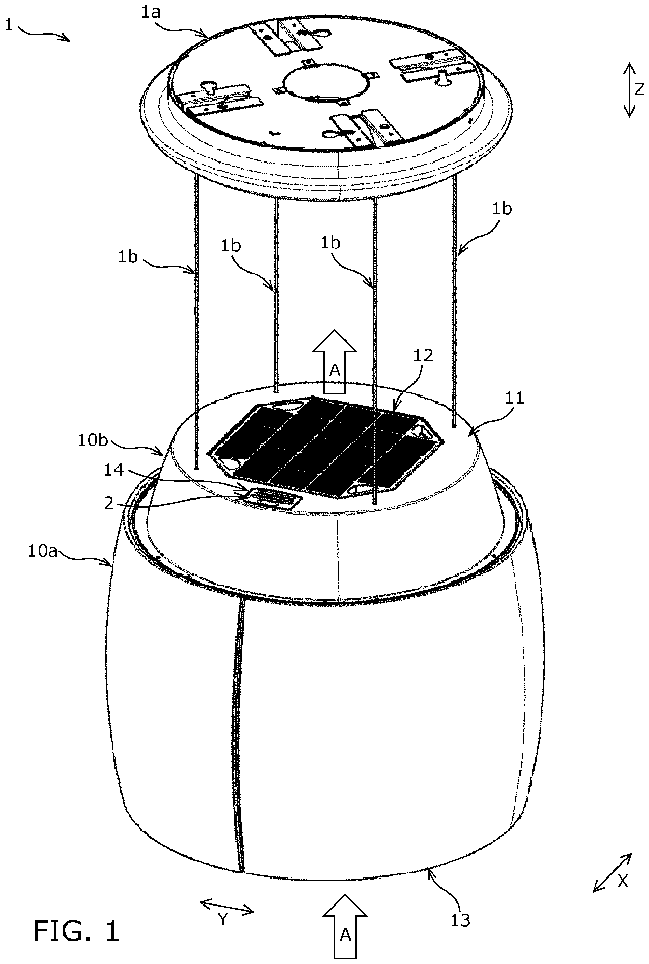

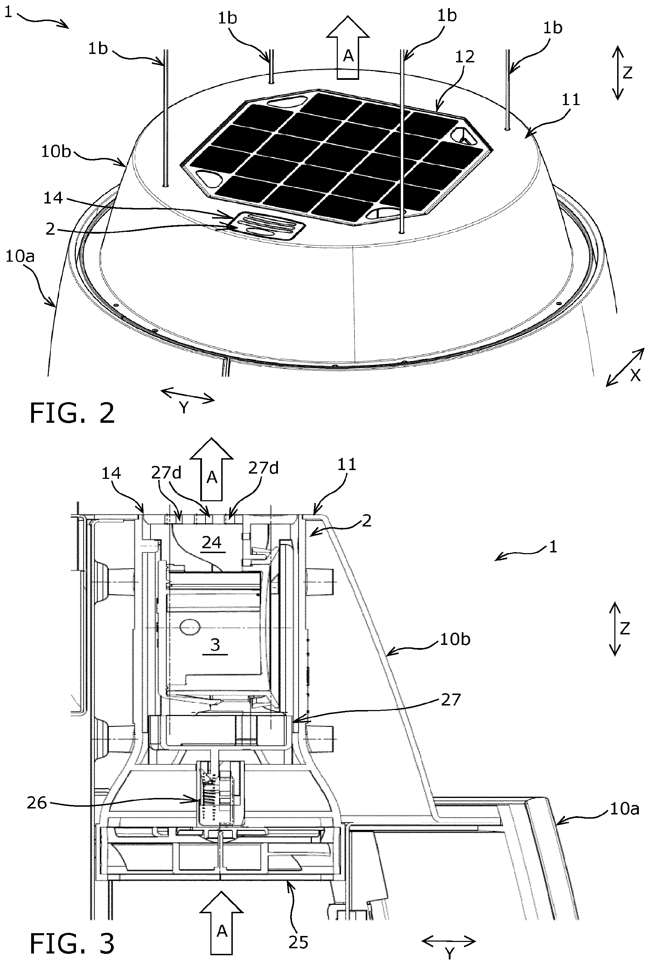

Fig. 1 zeigt eine perspektivische Darstellung einer erfindungsgemäßen Dunstabzugshaube 1. Fig. 2 zeigt eine Detailansicht der Fig. 1.

Erfindungsgemäß wird die Funktion einer Beduftungsvorrichtung, mittels ihrer Beduftungseinheit 2 einen Duftstoff an die Umgebungsluft abgeben zu können, in eine Dunstabzugshaube 1 integriert.

Eine erfindungsgemäße Dunstabzugshaube 1 ist in den Fig. 1 und 2 in Form einer deckenmontierbaren Dunstabzugshaube 1 dargestellt. Die Dunstabzugshaube 1 weist eine Deckenhalterung 1a auf, welche ausgebildet ist, in der Höhe Z von unten an einer Decke einer Küche oberhalb eines Kochfeldes montiert zu werden. An der Unterseite der Deckenhalterung 1a sind vier Haltemittel 1b in Form von vier Seilen 1b angeordnet, welche sich in der Höhe Z nach unten erstrecken.

Die eigentliche Dunstabzugshaube 1 weist einen unteren Gehäusemantel 10a sowie einen oberen Gehäusemantel 10b auf. Der untere Gehäusemantel 10a kann über Lufteinlässe (nicht dargestellt) seiner Unterseite 13 einen Luftstrom A aus dem Bereich des Kochfelds aufnehmen und mittels eines entsprechenden Lüfters der Dunstabzugshaube 1 durch sich hindurchbefördern. Im Bereich des oberen Gehäusemantels 10b ist eine entsprechende Filterung im Innern vorgesehen, welche Gerüche sowie Fette aus dem Luftstrom A herausfiltern kann. Der gefilterte Luftstrom A kann die Dunstabzugshaube 1 über dessen Oberseite 11 und dort vorgesehene Luftauslässe 12 wieder verlassen. Um die Luftauslässe 12 des oberen Gehäusemantels 10b herum verteilt werden die unteren Enden der vier Seile 1b aufgenommen, so dass die eigentliche Dunstabzugshaube 1 von den Seilen 1b sowie der Deckenhalterung 1a an einer Decke gehalten werden kann.

Erfindungsgemäß ist zusätzlich eine Aufnahme 14 für eine Beduftungseinheit 2 in der Oberseite 11 des oberen Gehäusemantels 10b vorgesehen. In der Aufnahme 14 wird die Beduftungseinheit 2 aufgenommen. Die Funktion der Beduftungseinheit 2 soll im Folgenden näher beschrieben werden.

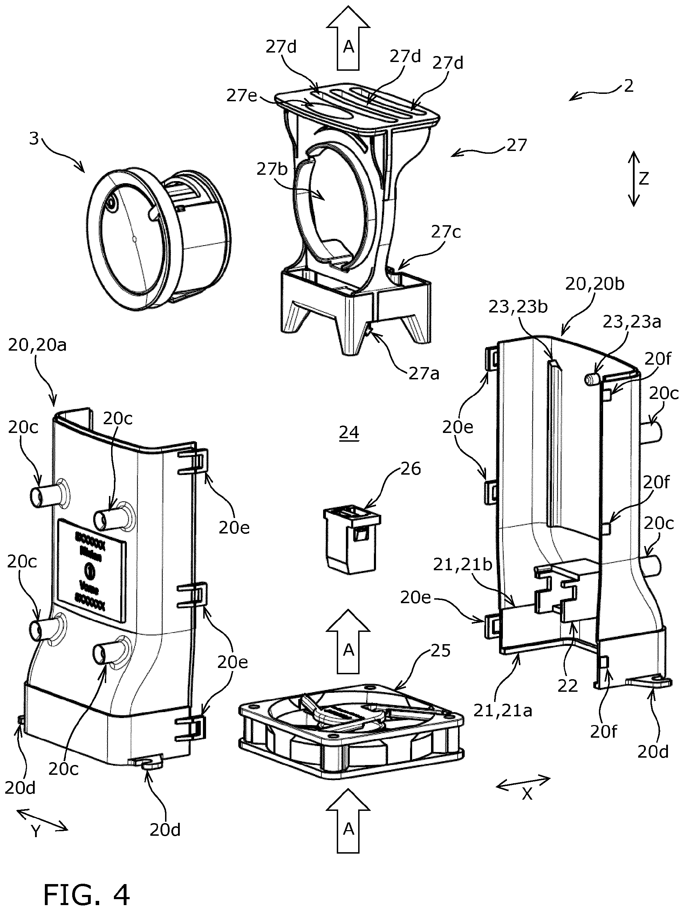

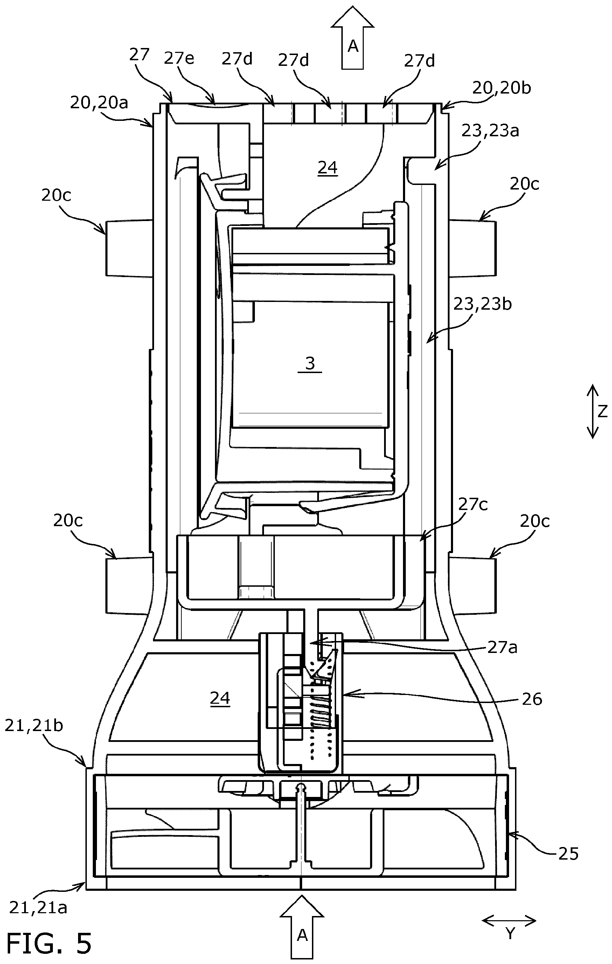

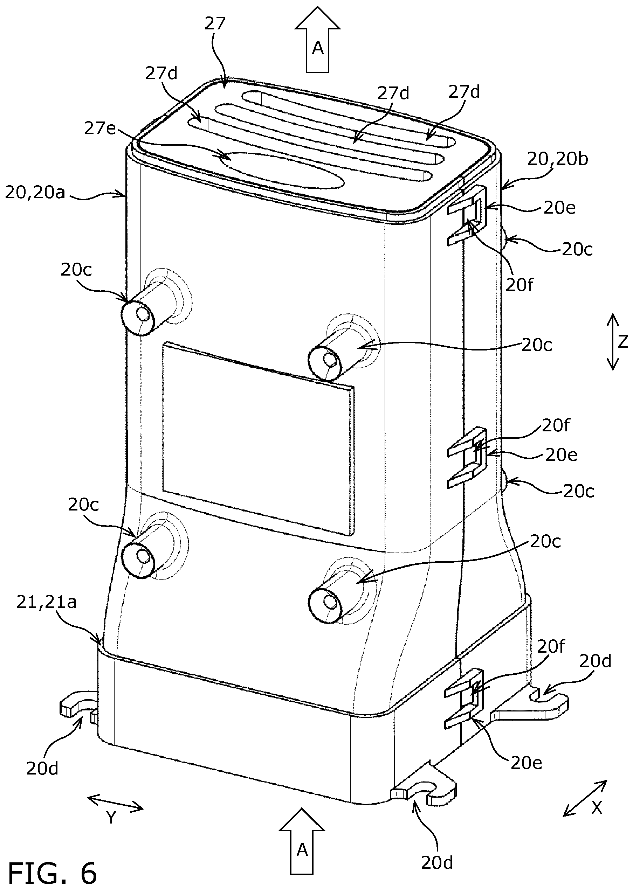

Fig. 3 zeigt einen Querschnitt einer Detailansicht der Fig. 2. Fig. 4 zeigt eine Explosionszeichnung der Beduftungseinheit 2 der erfindungsgemäßen Dunstabzugshaube 1. Fig. 5 zeigt einen Querschnitt der Beduftungseinheit 2 der erfindungsgemäßen Dunstabzugshaube 1. Fig. 6 zeigt eine perspektivische Darstellung der Beduftungseinheit 2 der erfindungsgemäßen Dunstabzugshaube 1.

Betrachtet man den Aufbau der Beduftungseinheit 2 am Beispiel der Explosionszeichnung der Fig. 4, so weist die Beduftungseinheit ein Gehäuse 20 auf, welches aus einem ersten Gehäuseelement 20a und aus einem zweiten Gehäuseelement 20b besteht. Entsprechend kann das erste Gehäuseelement 20a als erste Hälfte 20a des Gehäuses 20 und das zweite Gehäuseelement 20b als zweite Hälfte 20b des Gehäuses 20 bezeichnet werden. Die beiden Hälften 20a, 20b des Gehäuses 20 sind als Spritzgussteile jeweils einstückig ausgebildet. Die beiden Hälften 20a, 20b des Gehäuses 20 können zusammengefügt werden, um das einteilige Gehäuse 20 zu bilden.

In der Längsrichtung X weisen die beiden Hälften 20a, 20b des Gehäuses 20 an ihren Au-.ßenseiten jeweils vier Befestigungsmittelaufnahmen 20c in Form von Schraubenaufnahmen 20c auf, siehe Fig. 4 und 6. Diese sind jeweils nach außen hin gerichtet, so dass jeweils eine Schraube von einer Schraubenaufnahme 20c als Befestigungsmittel aufgenommen werden kann. Hierdurch kann das Gehäuse 20 in dem Gehäuse der Dunstabzugshaube 1 montiert werden, siehe z.B. Fig. 3.

Ferner weisen beide Hälften 20a, 20b des Gehäuses 20 in der Höhe Z im unteren Bereich jeweils zwei Halterungselemente 20d auf, welche sich in der Querrichtung Y gegenüberliegen. Hierdurch kann zur Vorbereitung der Montage ein vorläufiger Halt an dem Gehäuse der Dunstabzugshaube 1 erfolgen. Dieser Halt kann ausreichend sein, um das Gehäuse 20 vorzupositionieren, um in dieser Stellung das Gehäuse 20 mittels der Schrauben montieren zu können, wie zuvor beschrieben. Dies kann die Montage vereinfachen.

Die erste Hälfte 20a des Gehäuses 20 weist in der Querrichtung Y an ihrer linken sowie rechten Kante jeweils drei in der Höhe Z zueinander versetzt angeordnete Verbindungselemente 20e in Form von Rastelementen 20e auf. Die zweite Hälfte 20b des Gehäuses 20 weist in einer korrespondierenden Anordnung entsprechende Verbindungselemente 20f in Form von Hakenelementen 20f auf. Die Rastelemente 20e der ersten Hälfte 20a des Gehäuses 20 sowie die Hakenelemente 20f der zweiten Hälfte 20b des Gehäuses können miteinander verrastet werden, um die beiden Hälften 20a, 20b des Gehäuses miteinander kraftschlüssig und bzw. oder formschlüssig zu verbinden und hierdurch das einteilig Gehäuse 20 zu bilden.

Auf der Innenseite der beiden Hälften 20a, 20b des Gehäuses 20 ist eine Lüfteraufnahme 21 ausgebildet. Die Lüfteraufnahme 21 weist in der Höhe Z übereinander liegend eine erste untere Aufnahmekante 21a sowie eine zweite obere Aufnahmekante 21b auf. Der Bereich der Höhe Z zwischen den beiden Aufnahmekanten 21a, 21b ist in der Horizontalen breiter ausgebildet als der umgebende Bereich des Gehäuses 20. In diesem Bereich kann ein Lüfter 25 mit seinen seitlichen Kanten von den beiden Aufnahmekanten 21a, 21b des Gehäuses 20 aufgenommen und in der Höhe Z sowohl nach oben als nach unten gehalten werden. Der Lüfter 25, welcher auch als Gebläse 25 bezeichnet werden kann, dient der Erzeugung des Luftstroms A durch die Beduftungseinheit 2 hindurch.

An den Innenseiten der beiden Hälften 20a, 20b des Gehäuses 20 ist jeweils eine Aufnahme 22 für eine Halterungseinheit 26 ausgebildet. Die Aufnahme 22 wird dabei zur Hälfte von der ersten Hälfte 20a des Gehäuses 20 und zur zweiten Hälfte von der zweiten Hälfte 20b des Gehäuses 20 gebildet. Hierzu sind jeweils aufeinander hinzeigende Aufnahmehälften ausgebildet, welche zwischen sich die Halterungseinheit 26 aufnehmen können.

Die Halterungseinheit 26, welche auch als Push-Push-Verriegelungsmechanismus 26 bezeichnet werden kann, ist dazu ausgebildet, ein Rastelement 27a eines Duftstoffquellenhalters 27, wie im Folgenden näher erläutert werden wird, in der Höhe Z von oben aufzunehmen. Der Push-Push-Verriegelungsmechanismus 26 ist dabei ausgebildet, durch ein erstes Drücken in der Höhe Z von oben das Rastelement 27a aufzunehmen und zu halten sowie durch ein erneutes Drücken in der Höhe Z von oben dieses wieder freizugeben und in der Höhe Z nach oben zu drücken. Diese Funktionen werden im Folgenden näher erläutert werden.

Der zuvor bereits erwähnte Duftstoffquellenhalter 27 dient der Aufnahme einer Duftstoffquelle 3. Die Duftstoffquelle 3 stellt ein Behältnis dar, welches einen Duftstoff insbesondere als Duftstoffflüssigkeit in sich speichernd aufnehmen und z.B. über einen Docht an die Umgebungsluft abgeben kann. Die Duftstoffquelle 3 kann auch als Duftstoffbehälter 3, als Duftstoffkapsel 3 oder als Duftflakon 3 bezeichnet werden. Entsprechend kann der Duftstoffquellenhalter 27 auch als Duftstoffbehälterhalter 27, als Duftstoffkapselhalter 27 oder als Duftflakonhalter 27 bezeichnet werden.

Der Duftstoffquellenhalter 27 ist ausgebildet, innerhalb eines Luftführungsschachts 24, welcher durch die beiden Hälften 20a, 20b des Gehäuses 20 zwischen ihnen ausgebildet wird, in der Höhe Z beweglich angeordnet zu werden. Der Duftstoffquellenhalter 27 ist ferner dazu ausgebildet, aus dem Luftführungsschacht 24 von einem Benutzer in der Höhe Z nach oben hin entnommen zu werden. Der Duftstoffquellenhalter 27 weist eine Aufnahme 27b auf, welche dazu ausgebildet ist, die Duftstoffquelle 3 in sich aufzunehmen. Hierzu kann die Duftstoffquelle 3 in der Längsrichtung X in die Aufnahme 27b des Duftstoffquellenhalters 27 eingesetzt werden. In diesem Zustand kann der Duftstoffquellenhalter 27 in der Höhe Z von oben in den Luftführungsschacht 24 eingeführt werden, bis der Duftstoffquellenhalter 27 mit seinem Rastelement 27a in Form eines Rasthakens 27a in den Push-Push-Verriegelungsmechanismus 26 des Gehäuses 20 eingreift und durch Drücken seitens des Benutzers in der Höhe Z von oben dort verriegelt wird.

Um bei diesem Vorgang des Einführens des Duftstoffquellenhalters 27a in den Luftführungsschacht 24 des Gehäuses 20 eine bessere Führung der Bewegung in der Höhe Z zu erreichen, weist die zweite Hälfte 20b des Gehäuses 20 zum einen ein Führungselement 23 in Form eines Führungszapfens 23a auf, welches punktförmig nach innen ragend im oberen Bereich der zweiten Hälfte 20b des Gehäuses 20 angeordnet ist. Der untere Bereich des Duftstoffquellenhalters 27 weist eine korrespondierende Aufnahme 27c auf, welche dazu ausgebildet ist, den Führungszapfen 23a zu greifen und an diesem vorbeigeführt zu werden. Dadurch, dass der Führungszapfen 23a bzw. die Aufnahme 27c lediglich auf einer Seite des Gehäuses 20 bzw. des Duftstoffquellenhalters 27 angeordnet ist, kann eine Codierung für den Benutzer erfolgen, in welche Orientierung der Duftstoffquellenhalter 27 in den Luftführungsschacht 24 einzuführen ist.

Um diese Bewegung innerhalb des Luftführungsschachts 24 zu führen, ist zum anderen als weiteres Führungselement 23 ferner eine Führungsschiene 23b an derselben Innenseite der zweiten Hälfte 20b des Gehäuses 20 angeordnet. Auch weist der Duftstoffquellenhalter 27 eine entsprechende Aussparung auf, welche dazu ausgebildet ist, die Führungsschiene 23b zu umgreifen (nicht dargestellt). Dies kann eine geradlinige Bewegung in der Höhe Z sowohl beim Einsetzen als auch beim Entnehmen des Duftstoffquellenhalters 27 ermöglichen.

Oberhalb der Aufnahme 27b für die Duftstoffquelle 3 schließt der Duftstoffquellenhalter 27 mit mehreren Auslassöffnungen 27d in Form von Auslassschlitzen 27d ab. Hierdurch kann der Luftstrom A aus dem Luftführungsschacht 24 an die Umgebung abgegeben werden. Neben den Auslassöffnungen 27d ist auch ein Betätigungselement 27e in Form einer Fingermulde 27e angeordnet, welches es dem Benutzer ermöglichen kann, haptisch die Position des Duftstoffquellenhalters 27 zu erkennen und diesen drücken zu können, um mittels des Push-Push-Verriegelungsmechanismus 26 den Duftstoffquellenhalter 27 in der Höhe Z nach oben vordrücken zu lassen. Dies kann die Entnahme für den Benutzer vereinfachen.

Erfindungsgemäß kann somit ein Luftstrom A von dem Lüfter 25 erzeugt werden, welcher den Luftführungsschacht 24 der Beduftungseinheit 2 durchqueren kann. Dabei wird der Luftstrom A an der Duftstoffquelle 3 vorbeigeführt, so dass über den Docht der Duftstoffquelle 3 dessen Duftstoff an den Luftstrom A abgegeben werden kann. Diesen Duftstoff kann der Luftstrom A mit sich mitführen, wenn er durch die Auslassöffnungen 27d nach oben aus der Dunstabzugshaube 1 hervortritt, vgl. Fig. 1 und 2. Die Intensität des Lüfters 25 kann dabei über die Bedienelemente der Dunstabzugshaube 1 mitgesteuert werden (nicht dargestellt). Auf diese Weise kann beispielsweise in mehreren diskreten vorgegebenen Stufen der Luftstrom A mit unterschiedlicher Stärke erzeugt werden, um je nach Stärke des Luftstroms A mehr oder weniger Duftstoff aufzunehmen und mit sich mit in die Umgebung zu führen.

Um eine leere Duftstoffquelle 3 auszuwechseln, kann ein Benutzer haptisch das Betätigungselement 27e in Form der Fingermulde 27e auf der Oberseite 11 des oberen Gehäusemantels 10d der Dunstabzugshaube 1 ertasten und durch Herunterdrücken betätigen, so dass der Duftstoffquellenhalter 27 aus der Dunstabzugshaube 1 in der Höhe Z nach oben herausgedrückt wird. Den hervorragenden Duftquellenhalter 27 kann der Benutzer dann mit den Fingern greifen und entnehmen, um die Duftstoffquelle 3 einfach und bequem auszutauschen.

Mit frischer Duftstoffquelle 3 bestückt kann dann der Duftstoffquellenhalter27 wieder in den Luftführungsschacht 24 der Beduftungseinheit 2 eingeführt werden. Hierbei können die Führungszapfen 23a sowie die Führungsschiene 23b verhindern, dass der Duftstoffquellenhalter 27d in einer unzulässigen Orientierung in den Luftführungsschacht 24 eingeführt werden kann. Ist die richtige Positionierung und Orientierung des Duftstoffquellenhalters 27 gegenüber dem Luftführungsschacht 24 gefunden, kann der Benutzer den Duftstoffquellenhalter 27 in den Luftführungsschacht 24 einführen und eindrücken, so dass dieser mit seinem Rasthaken 27a von dem Push-Push-Verriegelungsmechanismus 26 aufgenommen und gehalten werden kann. In diesem Zustand schließt die Oberseite des Duftstoffquellenhalters 27 mit der Oberseite 11 des oberen Gehäusemantels 10b der Dunstabzugshaube 1 bündig ab. In diesem Zustand kann die Beduftungseinheit 2, wie zuvor beschrieben, bedient und genutzt werden.

### Bezugszeichenliste (Bestandteil der Beschreibung)

- A: Luftstrom bzw. dessen Strömungsrichtung
- X: Längsrichtung; Tiefe
- Y: Querrichtung; Breite
- Z: vertikale Richtung; Höhe

- 1: Dunstabzugshaube
- 1a: Deckenhalterung
- 1b: Haltemittel; Seile
- 10a: unterer Gehäusemantel
- 10b: oberer Gehäusemantel
- 11: Oberseite
- 12: Luftauslass
- 13: Unterseite
- 14: Aufnahme für Beduftungseinheit 2

- 2: Beduftungseinheit
- 20: Gehäuse der Beduftungseinheit 2
- 20a: erstes Gehäuseelement; erste Hälfte des Gehäuses 20
- 20b: zweites Gehäuseelement; zweite Hälfte des Gehäuses 20
- 20c: Befestigungsmittelaufnahme; Schraubenaufnahmen
- 20d: Halterungselemente
- 20e: Verbindungselemente; Rastelemente
- 20f: Verbindungselemente; Hakenelemente
- 21: Lüfteraufnahme des Gehäuses 20
- 21a: erste, untere Aufnahmekanten des Gehäuses 20
- 21b: zweite, obere Aufnahmekanten des Gehäuses 20
- 22: Aufnahme für Halterungseinheit 26
- 23: Führungselemente
- 23a: Führungszapfen
- 23b: Führungsschiene
- 24: Luftführungsschacht
- 25: Lüfter; Gebläse
- 26: Halterungseinheit; Push-Push-Verriegelungsmechanismus
- 27: Duftstoffquellenhalter; Duftstoffbehälterhalter; Duftstoffkapselhalter; Duftflaconhalter
- 27a: Rastelement; Rasthaken
- 27b: Aufnahme für Duftstoffquelle 3
- 27c: Aufnahme für Führungselement 23a; Nut für Führungszapfen 23a
- 27d: Auslassöffnungen; Auslassschlitze
- 27e: Betätigungselement, Fingermulde

- 3: Duftstoffquelle; Duftstoffbehälter; Duftstoffkapsel; Duftflacon

## Patentansprüche

1. Dunstabzugshaube (1)
mit wenigstens einer Beduftungseinheit (2), welche ausgebildet ist, wenigstens einen Duftstoff an die Umgebungsluft der Dunstabzugshaube (1) abzugeben,
**dadurch gekennzeichnet, dass**
die Beduftungseinheit (2) von einer Oberseite (11) der Dunstabzugshaube (1) zugänglich angeordnet ist.

2. Dunstabzugshaube (1) nach Anspruch 1, **dadurch gekennzeichnet, dass**
die Beduftungseinheit (2), vorzugsweise ein Lüfter (25) der Beduftungseinheit (2), ausgebildet ist, vom Benutzer mittels wenigstens eines Bedienelements der Dunstabzugshaube (1) bedient zu werden.

3. Dunstabzugshaube (1) nach Anspruch 1 oder 2, **dadurch gekennzeichnet, dass**
die Beduftungseinheit (2), vorzugsweise ein Lüfter (25) der Beduftungseinheit (2), ausgebildet ist, von der Dunstabzugshaube (1) elektrisch versorgt zu werden.

4. Dunstabzugshaube (1) nach einem der vorangehenden Ansprüche, **dadurch gekennzeichnet, dass**
die Beduftungseinheit (2) wenigstens einen Duftstoffquellenhalter (27) aufweist, welcher ausgebildet ist, wenigstens eine Duftstoffquelle (3) austauschbar aufzunehmen und in der Beduftungseinheit (2) zu halten.

5. Dunstabzugshaube (1) nach Anspruch 4, **dadurch gekennzeichnet, dass**
der Duftstoffquellenhalter (27) ausgebildet ist, von der Beduftungseinheit (2) entnehmbar aufgenommen zu werden.

6. Dunstabzugshaube (1) nach Anspruch 5, **dadurch gekennzeichnet, dass**
die Beduftungseinheit (2) einen Luftführungsschacht (24) aufweist, welcher ausgebildet ist, Umgebungsluft der Dunstabzugshaube (1) zugeführt zu bekommen, durch die Beduftungseinheit (2) hindurch zu führen und über wenigstens eine Auslassöffnung (27d), vorzugsweise über eine Mehrzahl von Auslassöffnungen (27d), an die Umgebung der Dunstabzugshaube (1) wieder abzugeben,
wobei der Duftstoffquellenhalter (27) ausgebildet ist, in dem Luftführungsschacht (24) aufgenommen zu werden,
wobei vorzugsweise die Auslassöffnung (27d) von dem Duftstoffquellenhalter (27) ausgebildet wird.

7. Dunstabzugshaube (1) nach Anspruch 5 oder 6, **dadurch gekennzeichnet, dass**
die Beduftungseinheit (2) wenigstens eine Halterungseinheit (26) aufweist, welche ausgebildet ist, den aufgenommenen Duftstoffquellenhalter (27) in der Beduftungseinheit (2) zu halten.

8. Dunstabzugshaube (1) nach Anspruch 7, **dadurch gekennzeichnet, dass**
die Halterungseinheit (26) von wenigstens einer Aufnahme (22) der Beduftungseinheit (2), vorzugsweise von wenigstens einer Aufnahme (22) eines Gehäuses (20) der Beduftungseinheit (2), aufgenommen wird,
wobei vorzugsweise das Gehäuse (20) wenigstens zwei Gehäuseelemente (20a, 20b), vorzugsweise genau zwei Gehäuseelemente (20a, 20b), aufweist, welche gemeinsam die Aufnahme (22) ausbilden und die Halterungseinheit (26) zwischen sich aufnehmen.

9. Dunstabzugshaube (1) nach Anspruch 7 oder 8, **dadurch gekennzeichnet, dass**
die Halterungseinheit (26) als Push-Push-Verriegelungsmechanismus (26) ausgebildet ist und dass
der Duftstoffquellenhalter (27), vorzugsweise an seiner Unterseite, wenigstens ein Rastelement (27a) aufweist, welches ausgebildet ist, von dem Push-Push-Verriegelungsmechanismus (26) gehalten zu werden,
wobei vorzugsweise der Duftstoffquellenhalter (27), vorzugsweise an seiner Oberseite, wenigstens ein Betätigungselement (27e), vorzugsweise wenigstens eine Fingermulde (27e), aufweist, welche ausgebildet ist, durch Betätigung durch einen Benutzer den Push-Push-Verriegelungsmechanismus (26) zu betätigen.

10. Dunstabzugshaube (1) nach einem der Ansprüche 5 bis 9, **dadurch gekennzeichnet, dass**
die Beduftungseinheit (2), vorzugsweise ein Gehäuse (20) der Beduftungseinheit (2), wenigstens ein Führungselement (23) aufweist, welches ausgebildet ist, den Duftstoffquellenhalter (27) beim Entnehmen und/oder beim Einsetzen zu führen,
wobei der Duftstoffquellenhalter (27) wenigstens eine Aufnahme (27c) für wenigstens ein korrespondierendes Führungselement (23) aufweist,
wobei vorzugsweise die Beduftungseinheit (2), vorzugsweise das Gehäuse (20) der Beduftungseinheit (2), wenigstens einen Führungszapfen (23a) als Führungselement (23) und/oder wenigstens eine Führungsschiene (23b) als Führungselement (23) aufweist.

11. Dunstabzugshaube (1) nach einem der vorangehenden Ansprüche, **gekennzeichnet durch**
wenigstens einen Lüfter (25), welcher ausgebildet und angeordnet ist, einen Luftstrom (A) durch die Beduftungseinheit (2), vorzugsweiseweise durch eine von einem Duftstoffquellenhalter (27) aufgenommene Duftstoffquelle (3), zu erzeugen,
wobei der Lüfter (25) vorzugsweise ein zusätzlicher Lüfter (25) der Beduftungseinheit (2) ist und die Dunstabzugshaube (1) einen Hauptlüfter aufweist.

12. Dunstabzugshaube (1) nach Anspruch 11, **dadurch gekennzeichnet, dass**
der Lüfter (25) innerhalb eines Gehäuses (20) der Beduftungseinheit (2) angeordnet ist,
wobei vorzugsweise der Lüfter (25) in der Strömungsrichtung des Luftstroms (A) wenigstens einseitig von einer Lüfteraufnahme (21) des Gehäuses (20) gehalten wird,
wobei vorzugsweise der Lüfter (25) in der Strömungsrichtung des Luftstroms (A) beidseitig von einer ersten Aufnahmekante (25a) des Gehäuses (20) und von einer zweiten Aufnahmekante (25b) des Gehäuses (20) gehalten wird.

13. Dunstabzugshaube (1) nach einem der vorangehenden Ansprüche, **dadurch gekennzeichnet, dass**
die Beduftungseinheit (2) ein Gehäuse (20) mit wenigstens zwei Gehäuseelementen (20a, 20b), vorzugsweise mit genau zwei Gehäuseelementen (20a, 20b), aufweist,
wobei die beiden Gehäuseelemente (20a, 20b) jeweils Verbindungselemente (20e, 20f) aufweisen, welche ausgebildet sind, die beiden Gehäuseelemente (20a, 20b) formschlüssig und/oder kraftschlüssig miteinander zu verbinden,
wobei vorzugsweise das erste Gehäuseelement (20a) wenigstens ein Rastelement (20e), vorzugsweise eine Mehrzahl von Rastelementen (20e), als Verbindungselement (20e) und das zweite Gehäuseelement (20b) wenigstens ein Hakenelement (20f), vorzugsweise eine Mehrzahl von Hakenelementen (20f), als Verbindungselement (20f) aufweist, welche ausgebildet sind, die beiden Gehäuseelemente (20a, 20b) formschlüssig und/oder kraftschlüssig miteinander zu verbinden.

14. Dunstabzugshaube (1) nach einem der vorangehenden Ansprüche, **dadurch gekennzeichnet, dass**
die Beduftungseinheit (2), vorzugsweise ein Gehäuse (20) der Beduftungseinheit (2), wenigstens eine Befestigungsmittelaufnahme (20c), vorzugsweise eine Mehrzahl von Befestigungsmittelaufnahmen (20c), aufweist, welche ausgebildet ist, wenigstens ein Befestigungsmittel aufzunehmen, so dass die Beduftungseinheit (2) von der Dunstabzugshaube (1) gehalten werden kann, und/oder dass
die Beduftungseinheit (2), vorzugsweise ein Gehäuse (20) der Beduftungseinheit (2), wenigstens ein Halterungselement (20d), vorzugsweise eine Mehrzahl von Halterungselementen (20d), aufweist, welches ausgebildet ist, so dass die Beduftungseinheit (2) von der Dunstabzugshaube (1) gehalten werden kann.
